# EUROPEAN PATENT APPLICATION

(11) **EP 1 806 670 A1**
(43) Date of publication of application: **11.07.2007**
(21) Application number: 07250063.0
(22) Date of filing: 08.01.2007
(51) Int. Cl.: G06F 19/00

(54) **A method and apparatus for processing patient information**

(30) Priority: 07.01.2006 GB 0600294
(71) Applicant: Safe Surgery Systems Limited, Bordesley Green East Birmingham B9 5SS (GB)
(72) Inventor: Morgan, David W., Birmingham B47JX (GB)
(74) Representative: Lockey, Robert Alexander

(57) **Abstract**

A method of processing patient information comprising the steps of physically attaching an electronic tag to the patient, obtaining patient data, and encoding the patient information data on the electronic tag.

## Description

### Description of Invention

This invention relates to a method of processing patient information and an apparatus for processing patient information, particularly but not exclusively for identifying victims of Mass Casualty Incidents (MCI's).

A Mass Casualty Incident can be defined as a disastrous event or other circumstance which results in a large number of fatalities or injuries and has the potential to exceed the logistical capacity of local health care providers. Such an incident can be a natural disaster such as an earthquake, a tornado, flooding etc, civil disorder, a plane crash, major fire and so on. To enable the available treatment and care capacity to be most efficiently used, it is known to carry out a process known as triage in which patients who are victims of an MCI can have their treatment needs assessed and be dealt with more efficiently. Essentially, triage aims to identify those who are seriously ill or injured as a direct result of the incident who require immediate treatment or care, particularly acute or emergency care, those affected by the incident who are not suffering any serious injury but will need assessment and treatment, and those who are neither ill nor injured but who still require information advice and reassurance.

Typically, the triage information consists of a paper form which a paramedic or other medical personnel who are at the site of the MCI completes with information relating to the patient's state. The triage process is typically performed at, or close to the MCI site. The form is then associated with the patient, accompanies the patient during transport to hospital and is used by the hospital staff to assist in deciding what acute care will be required. Typically, the document is passed off at the hospital upon arrival of the patient and when responsibility is formally handed over.

Clearly, during an incident, it is possible for the paper form to become damaged or lost, or even issued to the wrong patient. This can cause problems in assessing the care required by the patient, resulting in at least subsequent delays of treatment to that patient, in efficient use of resources, and could possibly put the patients safety at risk.

An aim of the present invention is to reduce or overcome one or more of the above problems.

According to a first aspect of the invention, we provide a method of processing patient information comprising the steps of physically attaching an electronic tag to the patient, obtaining patient data, and encoding the patient information data on the electronic tag.

The method may comprise the step of entering the data on a hand held device, wherein the step of encoding the data on the electronic tag comprises writing the data from the hand held device to the electronic tag.

The method may comprise the steps of obtaining location information indicating the location of the hand held device and writing the location information to the electronic tag.

An identifier may be associated with the electronic tag.

The method may comprise the step of transmitting the data to a receiving system.

The method may comprise the step of transmitting the identifier with the data.

The location information may also be transmitted to the receiving system.

The method may comprise subsequently modifying the data encoded on the electronic tag.

The method may comprise reading the data from the electronic tag and handling the patient accordingly.

The method may be performed as part of a triage process.

According to a second aspect of the invention, we provide an apparatus operable to perform a method according to the first aspect of the invention.

The hand held device may comprise a Personal Digital Assistant (PDA) or a tablet PC.

The electronic tag may comprise an RFID tag.

The RFID tag may be mounted on a wrist band to be attached to the patient's wrist.

The apparatus may include a GPS receiver.

The invention will now be described by way of example only with reference to the accompanying drawings, wherein;
Figure 1 is a diagrammatic illustration of an apparatus embodying the present invention,
Figure 2 is a diagrammatic illustration of a method embodying the present invention,
Figure 3 is a diagrammatic illustration of the method of Figure 2 included in a method of triage, and
Figure 4 is a diagrammatic illustration of a system using the apparatus of Figure 1.

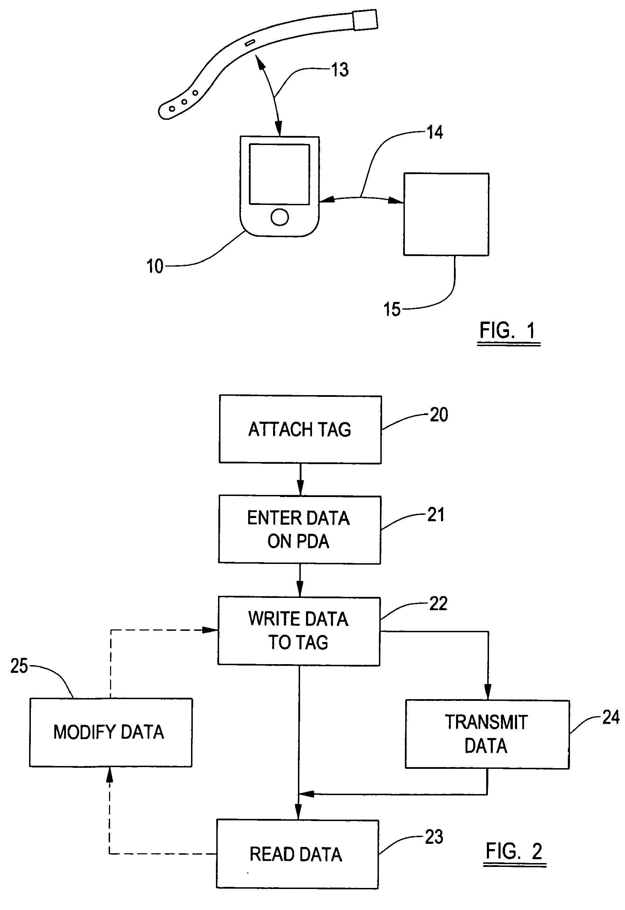

With reference to Figure 1, the apparatus comprises a hand held device 10, in the present example comprising a Personal Digital System (PDA), a wrist band 11 provided with an electronic tag 12 and a receiving system 15 associated with, for example, a hospital. In the present example, the electronic tag 12 comprises an RFID transponder. This for example can comprise a transponder which addressable at the frequency of 13.56 MHz and can store approximately 2kbits of data. The data held in the electronic tag 12 is non-volatile. As illustrated by arrow 13, the handheld device 10 is operable to communicate with the electronic tag 12 by an appropriate radio link to read from and write data to the electronic tag 12 when the handheld device 10 is sufficiently close the wrist band 11. Advantageously the hand held device 10 is provided with a small portable form factor, has a long battery life and a robust casing to enable the device to be used at an incident site.

As illustrated by arrow 14, the hand held device 10 is further operable to establish a communication link with the receiving system 15, for example through a GPRS or 3G wireless telephony link.

A method embodying the invention described with reference to Figure 2. At step 20, a tag is attached to a patient, for example by means of the wrist band 11 of Figure 1. Preferably the electronic tag 12 is initially blank (i.e. it contains no data) and has an associated identifier. At step 21, the paramedic or other attendant medical person obtains patient information in the form of triage data and enters the relevant triage data 21 on the hand held device 10, and at step 22, the data is written to the electronic tag 12 over link 13, providing that the hand held device 10 is physically close enough to the electronic tag 12. Subsequently the data can be read from the electronic tag 12 and as illustrated at step 23, when the patient is received at a hospital. To allow for advanced planning, as illustrated at 24 the data can also be transmitted from the hand held device 10 to the receiving system 15. Further, as shown at step 25, data held in the electronic tag 12 can be consequently be modified, for example to reflect care that the patient has received following the initial writing of data to the tag at step 21.

The triage data can contain such information as may be appropriate, for example the following:
- Patient's name, address and date of birth;
- Chief complaint information;
- Patient's relevant medical history, medication, allergies, etc;
- Cardiac, respiratory, circulation and trauma information;
- Consciousness and Glasgow Coma Scale information;
- Drugs administered at the scene;
- Other medical intervention at the scene.

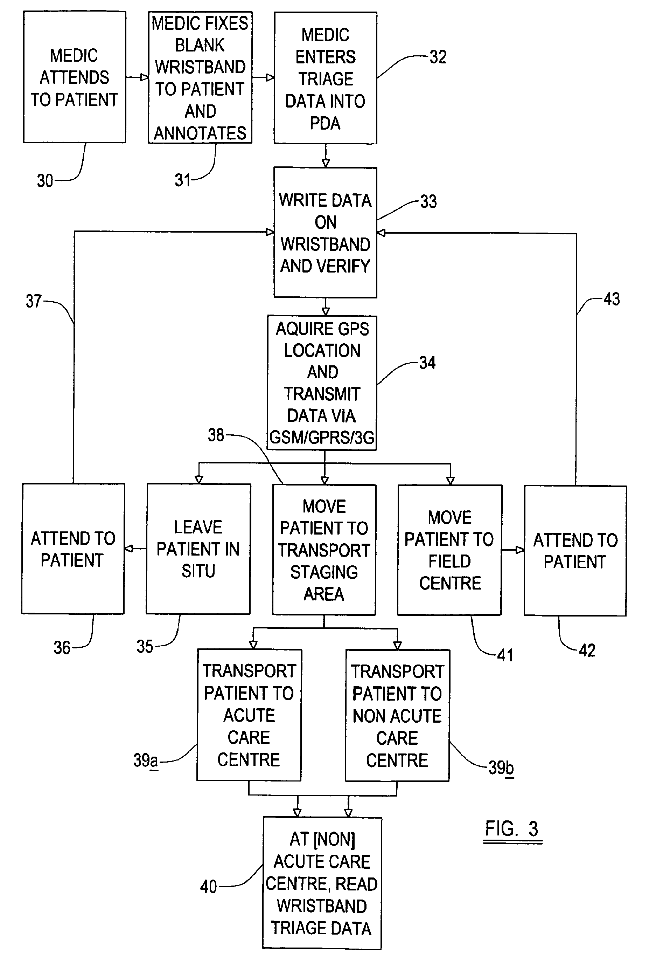

The use of the apparatus at Figure 1 and the method of Figure 2 in response to an MCI is illustrated with reference to Figure 3. At step 30, a paramedic or medical practitioner at the site of the MCI herein, referred to as a medic, attends to a patient who is a victim of the incident. At step 31, the medic fixes a blank wrist band 11 to a patient and may for example annotate the wrist band using a pen or other marking to give visual information if required. At step 32 the medic collects the triage information as listed above, and enters the data into the hand held device 10 as at step 21. At step 33, the handheld device 10 then writes the data to the electronic tag 12, and verifies the information, for example by reading the stored encoded data back from the electronic tag 12. At step 34, the hand held device 10 acquires its location, for example through the use of the Global Positioning System (GPS) and encodes this data on the electronic tag 12. The hand held device 10 also transmits the patient data, the location information and a unique identifier to the receiving system. The device 10 may also transmit the patient information and/or the location information to a receiving system, for example at a co-ordinating command, as desired in more detail below.

The subsequent triage steps depend on the assessment made by the medic attending to the patient. Where the patient can be left at the site as shown at step 35, the patient may subsequently be attended to at step 36 and, as shown by arrow 37, details of the subsequent treatment written to the electronic tag 12. If at step 38 it is decided to move the patient away from the site, then the patient is moved to a transport staging area and then sent to an acute care centre as shown at 39a or a non-acute care centre as shown at 39b. At the arrival at the care centre, the electronic tag 12 may be read to obtain the stored data, as shown at step 40. If the patient can be treated at a field centre close to the MCI site as shown at step 41, at step 42 the patient is attended to at the field centre. As shown by arrow 43, data reflecting the patient's treatment is written than to the electronic tag 12 and may also be transmitted to a necessary system. The patient's location information can be updated and transmitted to a receiving system.

The method and apparatus as described herein are thus advantageous in that they protect the integrity of the obtained patient information and ensures that it is not immediately separated from the patient. It protects patient confidentiality as the data held in electronic tag 12 can only be read by an appropriate person using a suitable hand held device 10, and indeed the data can be protected by requiring a password or other access restriction. Any suitable handheld device may be used, such as a tablet PC. The electronic tag may be attached to the patient in any other suitable way if desired, such as a neck band or ankle strap.

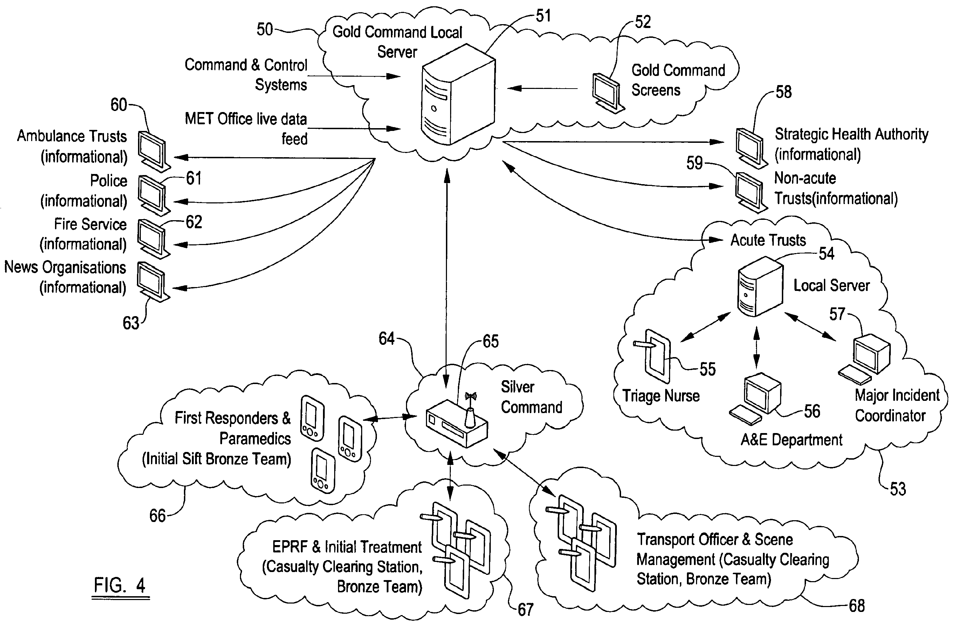

A system for responding to a mass casualty instrument which uses the apparatus is described by way of an example with reference to Figure 4. In Figure 4, a three tier major incident management system is shown. The top level of control is illustrated at 50, often referred to as "Gold Command". This is the top level of command and control and is responsible for co-ordinating the strategic allocation of resources for dealing with the incident. The command 50 may also make operational decisions such as to which hospitals patients should be sent. In this example, the Gold Command 50 has an infrastructure including a local server, illustrated at 51 which may be viewed on appropriate displays shown at 52. Gold Command 50 may also be responsible for receiving other data and communicating information to other organisations, particularly to a responding hospital shown at 53. In the example hospital 53, the hospital has a local server 54 which communicates the information to and from Gold Command 50 and in turn passes and receives information to and from a health care provider's apparatus 55, such as a PDA, the data system of a responding department shown at 56, such as the accident and emergency department, and the local co-ordinator shown at 57. The information may be supplied to other bodies, such as the local strategic health authority 58 and other local hospitals or organisations offering none acute care 59. Information may also be transmitted to other systems and organisations, such as the ambulance service, police, fire service and news organisations shown at 60, 61, 62 and 63 respectively.

Gold Command 50 communicates with the tactical control level, referred to as Silver Command and here illustrated at 64. The role of Silver Command 64 is to control operational resources at the site. Silver Command 64 will be provided with appropriate communication resources, illustrated at 65, preferably a wireless connection as illustrated here. At the operational level, are the teams responsible for the treatment of patients and their transfer to the hospital as appropriate. These are referred to as Bronze Teams, and may include a team of first responders and paramedics as shown at 66, a casualty clearing station as shown at 67, and a transport and site management team illustrated at 68. As shown, each of the members of the Bronze Teams 66, 67, 68 are provided with suitable hand held devices 10, such as the apparatus described herein to enable them to transmit the patient data to Silver Command 64 by suitable link, which is then transmitted to Gold Command 50.

Accordingly, the patient information obtained, stored using the apparatus 10, and written to the electronic tag 12 is also made available to a central command control operation, the Gold Command 50. The Gold Command 50 will be aware of hospital bed capacity information for paediatric, neurological, burns, general trauma, ITU and isolation patients, supplied by the hospital system 53 from the major incident co-ordinator 57. The allocation of hospital beds can be co-ordinated based on patients who have been sent to hospital by the transport officer 68 of the relevant Bronze Team 68. The lists of patients and the electronic data as encoded on the RFID tags can be aggregated, used to track the location of casualties and keep track of the numbers and statuses of the casualties. To facilitate transport of patients, Gold Command may also use other information, such as lists of transport access routes and rendezvous points for emergency service vehicles at the incident site, and weather information from an authoritative source, particularly important where a casualty incident includes a chemical or nuclear incident. The information allows Gold Command 50 to therefore instruct the Bronze Team 68 with the relevant transport officer as to where patients should be sent, using the transport information and hospital capacity and status information received from the hospitals. It will be apparent that by scanning the patient's electronic tag 12, associating that data with the position and sending the data to Gold Command, the position and status of individual patients can be easily updated and maintained, and resources efficiently allocated.

The safety of the patient themselves is improved and the chance of the patient information being lost causing a delay in treatment or the possibility of wrong treatment is reduced, and the method and apparatus also helps ensure efficient allocation of medical resources to the patient. As the wrist band 11 is suitably rugged and is physically attached to the patient, the chance of the information being lost is lowered, whilst the transmission of patient information to a receiving system such as a hospital can speed up the treatment if the patient and planning for the arrival of the patient, thus allowing efficient handling of the casualties from the MCI. By logging a physical location associated with each patient, should the patient be in an unsuitable condition to be moved immediately, the patient can then be subsequently found by an appropriate medical team using the supplied position information. If the victim is a fatality, it similarly allows subsequent recovery of the body. The stored position information from a number casualties of the incident can be used in subsequent investigation and forensic reconstruction of the incident.

When used in this specification and claims, the terms "comprises" and "comprising" and variations thereof mean that the specified features, steps or integers are included. The terms are not to be interpreted to exclude the presence of other features, steps or components.

The features disclosed in the foregoing description, or the following claims, or the accompanying drawings, expressed in their specific forms or in terms of a means for performing the disclosed function, or a method or process for attaining the disclosed result, as appropriate, may, separately, or in any combination of such features, be utilised for realising the invention in diverse forms thereof.

## Claims

1. A method of processing patient information comprising the steps of physically attaching an electronic tag to the patient, obtaining patient data, and encoding the patient information data on the electronic tag.

2. A method according to claim 1 comprising the step of entering the data on a handheld device, wherein the step of encoding the data on the electronic tag comprises writing the data from the handheld device to the electronic tag.

3. A method according to claim 1 or claim 2 further comprising the step of obtaining location information indicating the location of the handheld device and writing the location information to the electronic tag.

4. A method according to any one of claims 1 to 3 wherein an identifier is associated with the electronic tag.

5. A method according to any one of claims 1 to 4 comprising a step of transmitting the data to a receiving system.

6. A method according to claim 5 where dependent on claim 4 further comprising the step of transmitting the identifier with the data.

7. A method according to claim 5 or claim 6 wherein dependent directly or indirectly on claim 3 comprising the step of transmitting the location information to the receiving system

8. A method according to any one of the preceding claims further comprising subsequently modifying the data encoded on the electronic tag.

9. A method according to any one of the preceding claims comprising the step of subsequently reading the data from the electronic tag and handling the patient accordingly.

10. A method according to claim 8 or claim 9 comprising the step of updating the location information and transmitting the updated location information to a receiving system.

11. A method according to any one of the preceding claims wherein the method is performed as part of a triage process.

12. An apparatus to perform a method according to any one of the preceding claims.

13. An apparatus according to claim 12 wherein the handheld device comprises a Personal Digital Assistant (PDA) or a tablet PC.

14. An apparatus according to claim 12 or claim 13 wherein the electronic tag comprises an RFID tag.

15. An apparatus according to claim 14 wherein the RFID tag is mounted on a wrist band to be attached to the patient's wrist.

16. An apparatus according to any one of claims 12 to 15 wherein the apparatus includes a GPS receiver.
